# EUROPEAN PATENT SPECIFICATION

(11) **EP 1 133 352 B1**
(45) Date of publication and mention of the grant of the patent: **02.01.2003**
(21) Application number: 99947627.8
(22) Date of filing: 17.09.1999
(51) Int. Cl.: B01J 19/00

(54) **BIO-ASSAY TECHNIQUE**
VERFAHREN ZUR BIOANALYSE
TECHNIQUE DE BIODOSAGES

(30) Priority: 17.09.1998 GB 9820163
(43) Date of publication of application: 19.09.2001
(73) Proprietor: Smartbead Technologies Limited, Cambridge, Cambridgeshire CB2 1GE (GB)
(72) Inventor: DAMES, Andrew, Cambridge, Cambridgeshire CB4 1HU (GB); ENGLAND, James, Cambridge, Cambridgeshire CB4 6DG (GB); COLBY, Edward, Cambridge, Cambridgeshire CB1 3LR (GB)
(74) Representative: Brasnett, Adrian Hugh
(86) International application number: GB9903109
(87) International publication number: WO00016893

(56) References cited:
- EP-A- 0 395 300
- WO-A-96/24061
- WO-A-97/12680
- WO-A-99/02266
- DE-A- 2 416 652
- GB-A- 2 306 484
- US-A- 5 129 974
- RIGBY W R ET AL.: "An anodizing process for the production of inorganic microfiltration membranes" TRANSACTIONS OF THE INSTITUTE OF METAL FINISHING, vol. 68, August 1990 (1990-08), pages 95-98, XP002130485
- PATENT ABSTRACTS OF JAPAN vol. 1996, no. 08, 30 August 1996 (1996-08-30) & JP 08 102544 A (SATO YOICHI), 16 April 1996 (1996-04-16)
- LAURELL T ET AL: "ENHANCED ENZYME ACTIVITY IN SILICON INTEGRATED ENZYME REACTORS UTILIZING POROUS SILICON AS THE COUPLING MATRIX" SENSORS AND ACTUATORS B,CH,ELSEVIER SEQUOIA S.A., LAUSANNE, vol. 31, no. 3, 1 March 1996 (1996-03-01), pages 161-165, XP000630913 ISSN: 0925-4005

## Description

### Field of the Invention

This invention relates to the field of micromachined or micro fabricated coded substrates, particularly but not exclusively for use as a parallel bioassay technique.

### Background

Massively parallel bioassay tests are the enabling techniques that have made the majority of recent advances in genetics, screening and drug discovery possible. Thousands or millions of tests that previously were carried out one by one may now be combined into a single experiment yielding thousands or millions of results. The key to this process has been the development of techniques that allow the results of the many different tests to be separated from each other.

A number of existing techniques are described below. The techniques consist of labelling each of the constituent experiments in a manner that can be read after the experiment has completed. Labels used at present include the position of the experiment on the surface of a test chip and the fluorescent spectrum of a particle to which the experiment is bound.

Affymetrix's GeneChip probe array is a DNA sequence testing chip, where tens of thousands of different DNA probes are located at known points on a large 2D array. The fabrication process is described in, for example US 5,744,305 or US 5,143,854. Standard DNA hybridisation techniques are used in a chamber above the chip, and the test results are read out optically by the positions of fluorescent dots on the array (see, for example, US 5,578,832). The combinations of tests are pre-determined during the manufacture of the chip.

Luminex Corporation's FlowMetrix system makes use of coded microspheres, 6.5 µm in diameter. Each bead incorporates red and orange fluorophores to make up the code.
Eight different intensities are possible, allowing 64 different bead types. A green fluorescent marker is used for the probes. This system is described in US 5,736,330. The system has a relatively small number of codes, and requires complex, multi-wavelength optics on flow cytometers to read the codes and fluorescence of the test.

NanoGen has a semiconductor-based microchip array, APEX which is aimed specifically at DNA binding and sequencing experiments. NanoGen's chip is programmable by the end user with different arrays of DNA probes (see for example, US 5,605,662 and US 5,929,208).

There are a number of other particle or substrate-based assay techniques under the general heading of Combinatorial Chemistry. For examples, NO 96/24061 describes a library of tests using radio-frequency identification tags. WO 97/32892 describes a composite support for use a combinatorial chemistry substrate. GB 2306,484, describes two-part support particles for combinatorial chemistry.

EP-A-0395300 discloses a macroscopic thin film diagnostic device having a layer of anodised aluminium. US-A-5129974 discloses a 1mm square label with anodised layers.

### Summary of the Invention

This invention describes a system for carrying out massively parallel multiple bioassay tests in low-cost, fast and convenient manner. The scheme involved making a suspension (an assay) containing many thousands of different types of micro-machined coded labels (micro-labels), each code carrying a different biochemical test or probe. A first aspect of the invention provides a solid support for a biochemical assay according to claim 1. A second aspect of the invention provides a method of fabricating the supports according to claim 11.

An assay is constructed by mixing together suspensions of chosen sets of active micro-labels. Assays are customised to particular applications, independently of the original fabrication of the micro-labels.

The sample under test is marked with a fluorescent label and incubated with the assay. Only micro-labels with probes that bind to the fluorescent sample molecules will become fluorescent.

The micro-labels are fabricated from an anodisable material such as aluminium, initially deposited onto a planar substrate with soluble release layer. The metal surface is anodised before patterning. This allows the attachment of a wide range of biochemically active agents for use as highly selective probes.

Standard optical lithography and dry etching is used to pattern the aluminium into separate micro-labels. The code is stored on the micro-label as a series of holes using coding schemes similar to those found on bar codes. The biochemical probes may be attached to the surface either before or after the lithography step. The micro-labels are then released into an aqueous suspension. Each different micro-label code has a unique probe associated with it. Micro-labels with up to 100,000 different variants have been demonstrated.

A flow-based reader system, similar to a flow cytometer, draws through thousands of micro-labels per second, reading both the bar code and the result of the test. The test result is measured by the degree of fluorescence. An alternative planar reading system, in which the micro-labels are plated out onto a flat substrate, uses fluorescence microscopy and image processing to read the results of the tests.

### Brief Description of the Drawings

Embodiments of the invention will now be described, by way of example, with reference to the accompanying drawings, in which:
Figure 1 illustrates a single micro-label incorporating a transmission optical barcode,
Figure 2 illustrates a parallel bio-assay comprising two binding event experiments,
Figure 3 illustrates a wafer scale fabrication process for micro-labels,
Figure 4 illustrates a porous structure resulting from anodic oxidisation of an aluminium surface,
Figure 5 illustrates a flow through micro-label reading device,
Figure 6 illustrates the use of orthogonal interrogation beams to enable labels to be read regardless of rotation

### Detailed Description

### Micro-labels

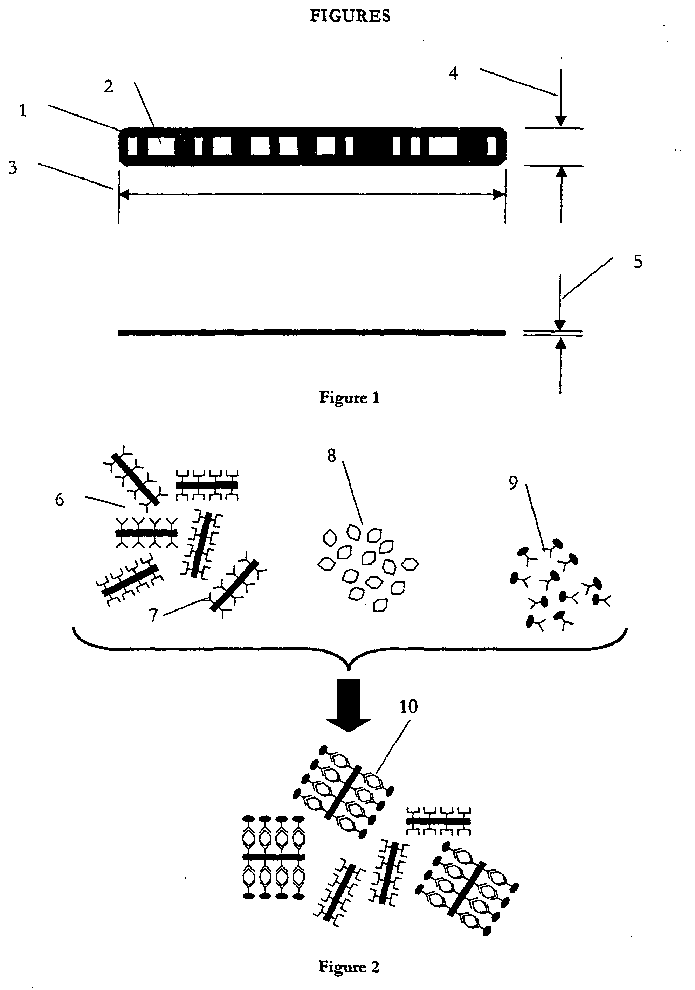

Figure 1 shows a micro-label, 1, in the form of a micro-machined miniature optical bar code, made from aluminium. The bar code is formed by a series of holes, 2, in the aluminium.

Each micro-label of this type is about 100 µm long, 3, by 10 µm wide, 4, by 1 µm thick, 5, and is capable of storing up to 100,000 different codes. Around 10 million such micro-labels can be fabricated on a single 6-inch diameter substrate. Different lengths of micro-label, from 40-100 µm, carrying between 2 and 5 decimal digits of data, have been fabricated. Each different code is associated with a unique biochemical probe. Coding schemes such as those used in EAN and UPC bar codes are used, to provide strong error checking when the codes are read.

### Probes and Assays

With reference to Figure 2, an assay, 6, of micro-labels, is constructed by mixing together suspensions of chosen sets of active micro-labels. Each different code has a unique biochemical probe associated with it, which binds to a specific type of molecule. Binding reactions may be selected from the group consisting of antibody-antigen, enzyme-substrate, enzyme-receptor, toxin-receptor, protein-protein and avidin-biotin.

The sample under test, 8, is marked with a fluorescent label and incubated with the assay. Only micro-labels, 7, with probes that bind to the fluorescent sample molecules will become fluorescent, 10. The result of the test is measured by the degree of fluorescence of different types of micro-labels.

An example of an application of the present invention is the screening of serurn for a selection of specific antibodies in a parallel bioassay. Antigens to the antibodies are used as probes and bound to micro-labels marked to identify these antigens. The micro-labels are incubated with the sample and then incubated with an antibody specific fluorescent label. The micro-labels are then passed though a reader that measures the degree of fluorescence of the labels and their identity. Correlation of the identities of the micro-labels with their degree of fluorescence provides indication of the binding of antibodies in the sample with the surface bound antigens.

### Micro-label Fabrication

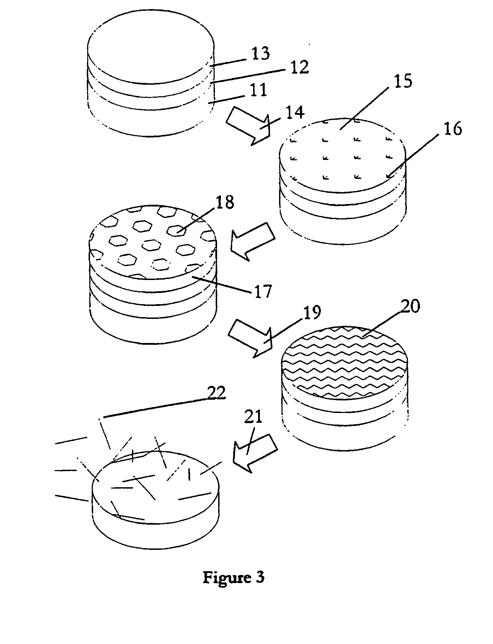

The fabrication process for the micro-labels will now be described with reference to Figure 3.

A substrate material, 11, such as a silicon wafer, is first coated with a soluble release layer, 12. In the preferred embodiment, this is a spin-coated layer of polymethyl methacrylate resist, baked at 150°C to drive off the solvents.

A layer of aluminium, 13, 1 µm thick, is deposited onto the substrate. This is achieved using a standard vacuum sputter coating technique, commonly used in semiconductor device fabrication.

The aluminium layer, 13, is anodised, 14, at a voltage of 30V in 4% phosphoric acid, using a pure aluminium cathode, for 30s, at a current density of 10 mA/cm². This leads to a 100 nm anodised layer, 15, with a pore size of around 40 nm.

At this stage (depending on the application) the aluminium surface may be coated with probe molecules, 16, by immersing the surface in an aqueous solution/suspension of the molecules.

The substrate is spin-coated with conventional optical resist, 17, Shipley S1813. The label pattern, 18, is exposed using a hard-contact optical mask, and developed using aqueous alkaline developer (MF319). The pattern is transferred into the aluminium layer using reactive ion etching with SiCl₄.

If the probe attachment is to be carried out after the lithography, the optical resist, 17, may be removed at this stage using a solvent such as isopropyl alcohol. This leaves the release layer, 13, intact, with the patterned aluminium layer, 20, on top. The probe molecules, 16, may then be attached as described above, whilst the micro-labels are still attached to the substrate.

The micro-labels with attached probes are released, 21, from the substrate using a solvent such as acetone. Dilution and filtration leaves the micro-labels, 22, in an aqueous suspension ready for combination into an assay.

The fabrication process is suitable for a wide variety of shapes of micro-labels with holes in, including both the substantially linear type of Figure 1 and other forms of square, rectangular and round micro-labels.

### Anodisation and Surface Chemistry

Proteins bind only weakly with an untreated aluminium surface when incubated in an aqueous solution. By modifying the surface this binding can be selectively enhanced to control when the binding occurs. This is important because it allows the probe molecules to be bound strongly to the surface at time of manufacture whilst maintaining weak nonspecific binding of the fluorescent target molecules during the test In this way the discrimination of the test is maximised.

There exists a very wide body of knowledge, gained in the first half of this century, on surface protection of aluminium components through the use of electrochemical processes. Methods for growing porous surfaces are well known, as are processes for sealing these surfaces. We have exploited this knowledge to develop a relatively simple process that grows an adsorbing surface with well controlled porosity and depth. This surface binds the chosen proteins well soon after treatment, but heals with time to prevent further binding.

In order to generate the appropriate anodised surface morphology, it is necessary to understand the structure of typical of biomolecules used as probes. Cryogenic atomic-force-microscopy allows direct imaging of such biomolecules. For example, Immunoglobulin-G (IgG) has a Y-shaped structure. The size of the IgG molecule is approximately 40nm across, which can be considered to be a typical size.

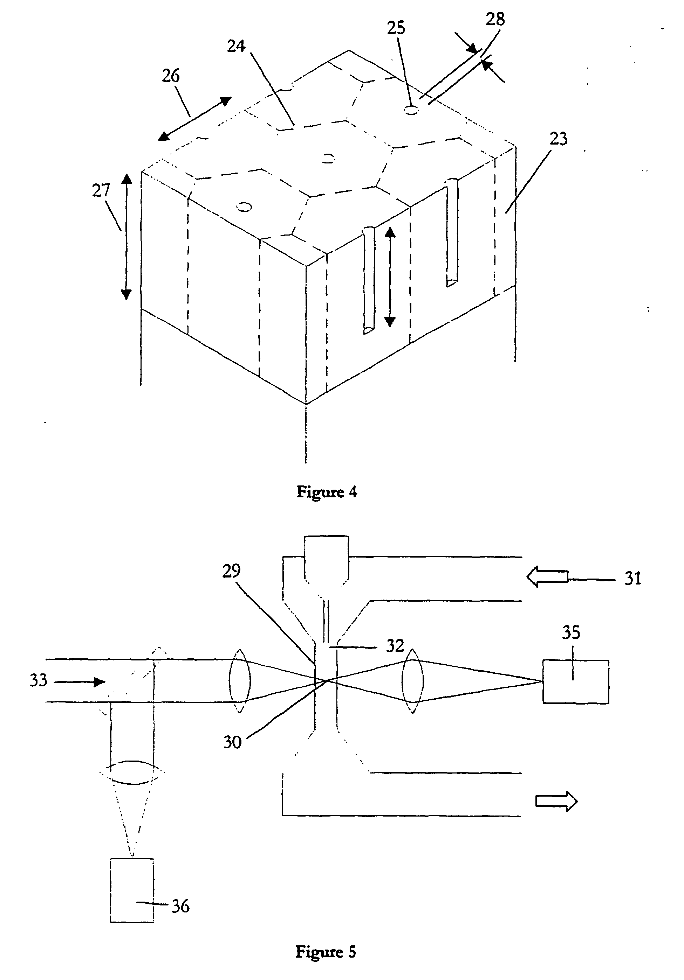

With reference to Figure 4, during anodisation, an aluminium oxide layer, 23, grows on the surface of the aluminium. This oxide layer grows in a two dimensional hexagonal cell structure, 24, the size of each cell depending on the electrochemical forming voltage. In the centre of each cell, a pore, 25, forms with an aperture smaller than the width of the cell, 26. As the anodisation process progresses, the thickness, 27, of the oxide layer and the depth of the pores, 28, increase, but the cell width, 26, and the pore aperture, 28, remains constant.

### Reading Systems

Two classes of reading system have been developed. These are based on flow cells, and on planar imaging of plated-out micro-labels.

The flow-cell reader, shown schematically in Figure 5, utilises a design that induces the micro-labels, 1, to flow down the centre of a tube, 29 through a defined reading zone, 30. Normally, elongated particles in flow would have a tendency to tumble. However, by using an accelerating sheath fluid, 31, and injecting the micro-labels into the centre of this flow, 32, a hydrodynamic focussing effect is achieved that causes all the micro-labels to align, and pass through a well-defined focal point, 30, somewhere downstream of the injection point, 32.

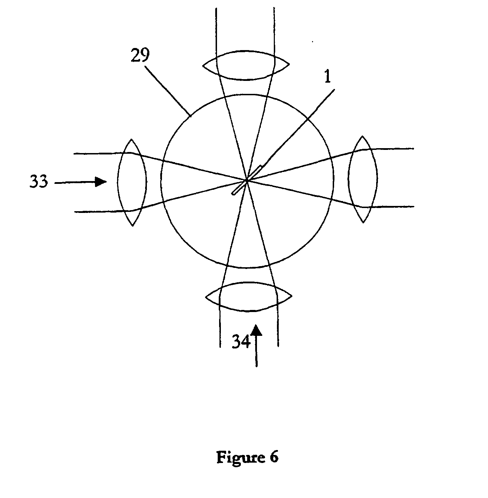

At the hydrodynamic focal point, 30, two orthogonal focused beams, 33,34, of laser light from a 488 nm argon-ion laser interrogate the micro-labels. This is detailed in Figure 6. The use of two orthogonal interrogation beams enables the interrogation of micro-labels, regardless of their rotation with respect to the frame of reference of the flow tube. The label, 1, in Figure 6, is shown at an angle of 45° to both incident beams, which is the worst possible case. The geometry of the micro-labels, shown in Figure 1, means that light is still transmitted through the holes in the label, 2. The holes modulate the transmitted intensity at the detector, 35, as the label passes through, generating a serial stream of information that is analysed in the same way as a conventional bar code. Simultaneously, the degree of fluorescence is measured, using an epi-fluorescence detector, 36. This is correlated with the code on the label. High numerical-aperture optics (microscope objective lenses) are used to achieve the desired resolution. The optimal flow-cell design has flat walls, to avoid the use of custom cylindrical imaging elements.

Once sufficient micro-labels have been read the reader calculates the results of all the tests. This number required is typically between 10 and 100 copies of each type of micro-label, to enable statistical analysis to be used. The test results show the mean and standard deviation of the fluorescence for each type of probe.

In a planar reading system, the micro-labels are plated out onto a flat substrate. This is either a filter substrate, or a transparent substrate. Conventional fluorescence microscopy is used to analyse the plated substrate systematically. An image-processing system captures pictures of each field of the substrate. Transmitted light is analysed separately from fluorescent light. Each micro-label is identified from its transmitted light profile, and the fluorescence signal integrated over the surface of the label is recorded. Once again, between 10 and 100 of each type of micro-label are required to give a good statistical analysis.

### Applications

A typical application uses a suspension of probes for a number of different antibodies (e.g. hepatitis, HIV). A reading system takes a drop of blood from a patient, labels it with fluorescent marker, and incubates it with the probe suspension for a few minutes. The suspension is fed through a micro fluidic detector system.

### Further Embodiments

Micro-label designs for planar reading systems are not limited to the linear designs described above, which are primarily intended for flow-through reading systems. Planar systems can use 2D patterns, fabricated on labels which are closer to being square or rectangular, rather than linear. Coding schemes similar to conventional 2D barcodes are then used. The outline of the label also gives information, particularly about orientation.

Labels incorporating magnetic material can use magnetic separation. This is useful if the sample being tested contains solid matter of similar size to the labels that could contaminate the results.

Micro-labels can be fabricated using any substrate that can be coated with an anodisable metal such as aluminium. This is particularly attractive when a mass-production method such as embossing of an aluminium-coated plastic is used to fabricate the micro-labels.

## Claims

1. A solid support (1, 22) for a biochemical assay, which support is substantially linear or planar in shape and has an anodised metal surface layer (13), **characterised in that** the largest dimension of the support is less than 100µm, whereby an aqueous suspension is formable from a plurality of the supports.

2. A support according to claim 1, wherein the surface layer has a cellular structure anodisation layer (15, 23), the growth direction of the cells of the anodisation layer being perpendicular to the plane of the surface layer.

3. A support according to claim 1 or 2, wherein probe molecules (16) for the biochemical assay are bound to the surface layer.

4. A support according to any one of claims 1 to 3, wherein the surface layer is of aluminium.

5. A support according to any one of claims 1 to 4, wherein the surface layer is porous.

6. A support according to claim 5, wherein the pore size of the surface layer is approximately matched to the size of the biochemically active molecules to be bound.

7. A support according to any one of claims 1 to 6, incorporating a spatially varying pattern (18) for identification purposes.

8. A support according to claim 7, wherein said pattern is a barcode.

9. A support according to claim 8, wherein the barcode is a linear barcode.

10. A support according to any one of claims 7 to 9, in which the pattern comprises a series of holes (2) in the support.

11. A method of fabricating the supports of one of claims 1 to 10, comprising sputter coating a flat surface with a metal layer (13), anodising the metal layer, and lithographically patterning and etching the metal layer to reveal the supports.

12. A method according to claim 11, wherein said surface consists of a layer of soluble material (12) on a rigid substrate (11), and the method further comprises releasing the supports from said surface by solvation of the soluble material.

13. A method according to claim 12, wherein the soluble material is a resist.

14. A method according to any one of claims 11 to 13, wherein the anodising is carried out at a voltage of up to 150 V.

15. A method according to claim 14, wherein the anodising is carried out at a voltage in the range from 4 V to 30V.

16. A method according to any one of claims 11 to 15, further comprising binding probe molecules (16) to the anodised metal layer.

## Patentansprüche

1. Fester Träger (1, 22) für einen biochemischen Test, wobei der Träger eine im Wesentlichen lineare oder planare Form besitzt und eine eloxierte Metalloberflächenschicht (13) aufweist, **dadurch gekennzeichnet, dass** die größte Abmessung des Trägers unter 100 µm beträgt, wodurch aus einer Vielzahl der Träger eine wässrige Suspension gebildet werden kann.

2. Träger nach Anspruch 1, worin die Oberflächenschicht eine eloxierte Schicht (15, 23) mit Zellenstruktur ist, wobei die Wachstumsrichtung der Zellen der eloxierten Schicht im rechten Winkel zur Ebene der Oberflächenschicht verläuft.

3. Träger nach Anspruch 1 oder 2, worin Sondenmoleküle (16) für den biochemischen Test an die Oberflächenschicht gebunden sind.

4. Träger nach einem der Ansprüche 1 bis 3, worin die Oberflächenschicht aus Aluminium besteht.

5. Träger nach einem der Ansprüche 1 bis 4, worin die Oberflächenschicht porös ist.

6. Träger nach Anspruch 5, worin die Porengröße der Oberflächenschicht ungefähr an die Größe der zu bindenden biochemisch aktiven Moleküle angepasst ist.

7. Träger nach einem der Ansprüche 1 bis 6, der ein räumlich variierendes Muster (18) für Identifizierungszwecke umfasst.

8. Träger nach Anspruch 7, worin das Muster ein Strichcode ist.

9. Träger nach Anspruch 8, worin der Strichcode ein linearer Strichcode ist.

10. Träger nach einem der Ansprüche 7 bis 9, bei dem das Muster eine Abfolge von Löchern (2) im Träger umfasst.

11. Verfahren zur Herstellung von Trägern nach einem der Ansprüche 1 bis 10, umfassend Sputterbeschichtung einer ebenen Oberfläche mit einer Metallschicht (13), Eloxieren der Metallschicht und lithographische Musterzeichnung und Ätzen der Metallschicht, um die Träger freizulegen.

12. Verfahren nach Anspruch 11, worin die Oberfläche aus einer Schicht aus löslichem Material (12) auf einem steifen Substrat (11) besteht und das Verfahren weiters das Ablösen der Träger von der Oberfläche durch Solvatisierung des löslichen Materials umfasst.

13. Verfahren nach Anspruch 12, worin das lösliche Metall ein Resist ist.

14. Verfahren nach einem der Ansprüche 11 bis 13, worin das Eloxieren bei einer Spannung von bis zu 150 V erfolgt.

15. Verfahren nach Anspruch 14, worin das Eloxieren bei einer Spannung im Bereich von 4 V bis 30 V erfolgt.

16. Verfahren nach einem der Ansprüche 11 bis 15, weiters umfassend das Binden von Sondenmolekülen (16) an die eloxierte Metallschicht.

## Revendications

1. Support solide (1, 22) pour un dosage biochimique, lequel support est sensiblement linéaire ou plan en forme et a une couche de surface de métal anodisé (13), caractérisé en que la plus grande dimension du support est plus petite que 100 µm, et une suspension aqueuse peut être formée d'un certain nombre de supports.

2. Support selon la revendication 1, où la couche de surface a une couche d'anodisation à structure cellulaire (15, 13), la direction de croissance des cellules de la couche d'anodisation étant perpendiculaire au plan de la couche de surface.

3. Support selon la revendication 1 ou 2, où des molécules de sonde (16) pour le dosage biochimique sont liées à la couche de surface.

4. Support selon l'une quelconque des revendications 1 à 3, où la couche de surface est en aluminium.

5. Support selon l'une quelconque des revendications 1 à 4, où la couche de surface est poreuse.

6. Support selon la revendication 5 où la dimension des pores de la couche de surface est à peu près adaptée à la dimension des molécules biochimiquement actives à lier.

7. Support selon l'une quelconque des revendications 1 à 6, incorporant un motif variant dans l'espace (18) dans des buts d'identification.

8. Support selon la revendication 7, où ledit motif est un code barre.

9. Support selon la revendication 8, où le code barre est un code barre linéaire.

10. Support selon l'une quelconque des revendications 7 à 9, où le motif comprend une série de trous (2) dans le support.

11. Méthode de fabrication des supports de l'une des revendications 1 à 10, comprenant le revêtement par pulvérisation d'une surface plate avec une couche de métal (13), l'anodisation de la couche de métal et la formation d'un motif lithographique et l'attaque de la couche de métal pour révéler les supports.

12. Méthode selon la revendication 11, où ladite surface consiste en une couche d'un matériau soluble (12) sur un substrat rigide (11), et la méthode comprend de plus la libération des supports de ladite surface par solvatisation du matériau soluble.

13. Méthode selon la revendication 12, où le matériau soluble est un résist.

14. Méthode selon l'une quelconque des revendication 11 à 13 où l'anodisation est effectuée à une tension pouvant atteindre 150 V.

15. Méthode selon la revendication 14 où l'anodisation est effectuée à une tension dans la gamme de 4 V à 30 V.

16. Méthode selon l'une quelconque des revendications 11 à 15, comprenant de plus la liaison des molécules de sonde (16) à la couche de métal anodisé.
